# EUROPÄISCHE PATENTANMELDUNG

(11) **EP 1 127 874 A2**
(43) Veröffentlichungstag der Anmeldung: **29.08.2001**
(21) Anmeldenummer: 01103687.8
(22) Anmeldetag: 23.02.2001
(51) Int. Cl.: C07D 209/08, C07D 209/12, C07D 209/14, C07D 409/04, C07D 417/04, C07D 471/04, C07D 487/04, C07D 471/14

(54) **Verfahren zur Herstellung substituierter Indole**

(30) Priorität: 25.02.2000 DE 10009000
(71) Anmelder: BASF AKTIENGESELLSCHAFT, 67056 Ludwigshafen (DE)
(72) Erfinder: Henkelmann, Jochem, Dr., 68165 Mannheim (DE); Arndt, Jan-Dirk, Dr., 68167 Mannheim (DE)
(74) Vertreter: Isenbruck, Günter, Dr.

(57) **Zusammenfassung**

Die vorliegende Anmeldung beschreibt ein Verfahren zur Herstellung von Verbindungen des Indoltyps der allgemeinen Formel 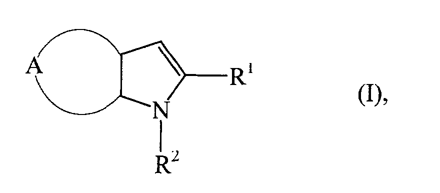 in der A einen Kohlenwasserstoffrest bedeutet, der gemeinsam mit den Kohlenstoffatomen, an die er gebunden ist, ein substituiertes oder unsubstituiertes, ein- oder mehrkerniges aromatisches System darstellt, das eines oder mehrere Heteroatome aus der Gruppe von N, O, S enthalten kann, und
R¹, R² unabhängig voneinander H, einen gesättigten, linearen oder verzweigten aliphatischen C₁-C₂₀-Kohlenwasserstoffrest, einen ungesättigten, linearen oder verzweigten aliphatischen C₂-C₂₀-Kohlenwasserstoffrest, einen gesättigten oder ungesättigten, gegebenenfalls alkylsubstituierten cycloaliphatischen C₃-C₂₀-Kohlenwasserstoffrest oder einen aromatischen C₅-C₂₀-Kohlenwasserstoffrest darstellen, wobei diese Reste eines oder mehrere Heteroatome aus der Gruppe bestehend aus Halogenen, N, P, O, S, Si, Sn und B in ihrem Molekülgerüst enthalten und substituiert oder unsubstituiert sein können,
durch Cyclisierung von Alkynylaminoaromaten der allgemeinen Formel 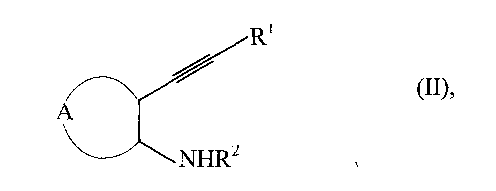 in der R¹ und R² die in Formel (I) angegebene Bedeutung aufweisen, und R¹, R² oder A gegebenenfalls mit einem organischen oder anorganischen Träger verbunden sind,
und wobei die Reaktion in einem polaren aprotischen, ionensolvatisierenden Lösungsmittel in Anwesenheit einer geeigneten Verbindung von Na, K, Rb oder Cs durchgeführt wird.

Mit diesem Verfahren können substituierte Indole einfach und in hohen Ausbeuten hergestellt werden.

## Beschreibung

Die vorliegende Erfindung betrifft ein Verfahren zur Herstellung von substituierten Indolen der Formel (I), bei dem substituierte 2-Alkynylaniline in einem polaren aprotischen Lösungsmittel mit Hilfe einer geeigneten Alkalimetallverbindung cyclisiert werden.

Einfache und kostengünstige Indolsynthesen sind von allgemeinem, großem Interesse. Dies liegt daran, daß das Strukturelement des Indols sich in zahlreichen Naturstoffen und insbesondere in pharmakologisch aktiven Substanzen wiederfindet. Bis jetzt existieren mehrere prinzipielle Routen, die den Zugang zu den gewünschten Indolderivaten eröffnen.

Die intramolekulare Addition von Aminogruppen einer Kohlenstoff-Kohlenstoff-Dreifachbindung unter Ausbildung von Indolderivaten ist eine literaturbekannte Reaktion. Beispielsweise kann sie durch Palladium-Komplexe katalysiert werden. Dies ist unter anderem in der Veröffentlichung von K. Utimoto et al. in Tetrahedron Letters 29, Seiten 3915ff(1992) beschrieben.

Andere Veröffentlichungen offenbaren die Verwendung von Molybdän(0)-Verbindungen in diesen Cyclisierungsreaktionen. Dies findet sich in den Publikationen von F. E. McDonald et al. in Tetrahedron Letters 38, Seiten 7687ff (1997) und in Chem. Eur. J. 5, Seiten 3103ff (1999).

In einer Variante, deren Beschreibung sich in mehreren Veröffentlichungen von S. Cacchi et al. findet, beispielsweise in Tetrahedron Letters 33, Seiten 3915ff (1992), Synlett 1997, Seiten 1393ff, Tetrahedron Letters 50, Seiten 437ff (1994), können dabei statt der Alkynylaniline die entsprechenden Trifluoracetamide eingesetzt werden, wobei als Katalysator auch hier Palladium-Komplexe eingesetzt werden.

Die Synthese substituierter Indole wird weiterhin von Yamanaka et al. in Heterocycles 24, Seiten 31/32 (1986) beschrieben. Dabei werden Alkinylcarbanilate in Anwesenheit von Na-Ethoxylat cyclisiert und die N-gebundene-C(O)O-Alkyl-Einheit durch Verseifung entfernt. Diese Cyclisierungsreaktion funktioniert nicht, wenn 2-Alkinylaniline als Edukt eingesetzt werden.

Bei allen diesen Reaktionen ist es jedoch häufig nötig, das Reaktionsgemisch auf relativ hohe Temperaturen zu erhitzen, oft sind auch zusätzlich lange Reaktionszeiten zum Erreichen annehmbarer Ausbeuten nötig. Durch diese generell notwendigen, scharfen Reaktionsbedingungen wird unter anderem die Bandbreite bezüglich des Einsatzes verschiedener Funktionalitäten in den 2-Alkynylanilinen stark eingeengt.

Es ist die Aufgabe der vorliegenden Erfindung, ein Verfahren bereitzustellen, mit dem substituierte Indole einfach und mit guten Ausbeuten sowie unter Anwendung kurzer Reaktionszeiten und niedriger Reaktionstemperaturen hergestellt werden können. Das Verfahren soll es weiterhin gestatten, verschiedene substituierte Indole mit einer großen Anzahl unterschiedlicher Substituenten darzustellen.

Diese Aufgabe wird gelöst durch ein Verfahren zur Herstellung von Verbindungen des Indoltyps der allgemeinen Formel 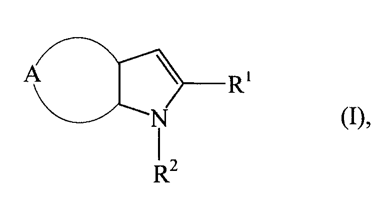 in der A einen Kohlenwasserstoffrest bedeutet, der gemeinsam mit den Kohlenstoffatomen, an die er gebunden ist, ein substituiertes oder unsubstituiertes, ein- oder mehrkerniges aromatisches System darstellt, das eines oder mehrere Heteroatome aus der Gruppe von N, O und S enthalten kann, und R¹, R² unabhängig voneinander H, einen gesättigten, linearen oder verzweigten aliphatischen C₁-C₂₀-Kohlenwasserstoffrest, einen ungesättigten, linearen oder verzweigten aliphatischen C₂-C₂₀-Kohlenwasserstoffrest, einen gesättigten oder ungesättigten, gegebenenfalls alkylsubstituierten cycloaliphatischen C₃-C₂₀-Kohlenwasserstoffrest oder einen aromatischen C₅-C₂₀-Kohlenwasserstoffrest darstellen, wobei diese Reste eines oder mehrere Heteroatome aus der Gruppe bestehend aus Halogenen, N, P, O, S, Si, Sn und B in ihrem Molekülgerüst enthalten und substituiert oder unsubstituiert sein können,
durch Cyclisierung von Alkynylaminoaromaten der allgemeinen Formel 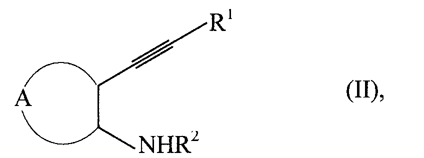 in der R¹ und R² die in Formel (I) angegebene Bedeutung aufweisen, und R¹, R² oder A gegebenenfalls mit einem organischen oder anorganischen Träger verbunden sind,
und wobei die Reaktion in einem polaren aprotischen, ionensolvatisierenden Lösungsmittel in Anwesenheit einer geeigneten Verbindung von Na, K, Rb oder Cs durchgeführt wird.

Es wurde gefunden, daß das erfindungsgemäße Verfahren den Zugang zu einer großen Menge an Verbindungen des Indoltyps erlaubt, in einer Bandbreite, die mit den bisher bekannten Verfahren nicht möglich war. Durch die Verwendung der Alkalimetallverbindungen, die im erfindungsgemäßen Verfahren ihren Einsatz finden, ist es möglich, Indolderivate darzustellen, die praktisch alle gängigen Substituenten R¹, R² aufweisen können. Die Zugänglichkeit bestimmter substituierter Indolderivate richtet sich dabei im Einzelfall nach dem Einfluß bestimmter Parameter. Dies sind beispielsweise die sterische Wechselwirkung zwischen einzelnen vorhandenen Substituenten und eventuell auch dem aromatischen System des Indolderivats. Die Bandbreite der unterschiedlichen Substituenten R¹ und R² sowie der Substituenten, die an dem aromatischen System der Formel (I) vorliegen können, ist enorm groß und umfaßt praktisch sämtliche Verbindungsklassen und funktionelle Gruppen, die unter die oben gegebene Definition fallen.

In einer bevorzugten Ausführungsform der vorliegenden Erfindung sind die Substituenten R¹ und R² unabhängig voneinander ausgewählt aus der Gruppe bestehend aus H, linearen und verzweigten C₁-C₁₂-Alkylgruppen, linearen und verzweigten C₂-C₁₂-Alkenylgruppen. C₃-C₈-Cycloalkylgruppen, C₃-C₈-Cycloalkenylgruppen, C₅- und C₆-Heterocyclen mit einem oder mehreren Atomen ausgewählt aus der Gruppe bestehend aus N, O und S im Ring und ein- oder zweikernigen Aromaten mit einem oder mehreren Atomen ausgewählt aus N, O und S im Ring.

Die Substituenten R¹ und R² können sowohl in den bevorzugten als auch in den nicht bevorzugten Ausführungsformen einen oder mehrere Substituenten an ihrem Molekülgerüst aufweisen.

Beispiele für bevorzugte Substituenten sind Amino- und Nitrogruppen, Halogene, Hydroxyl- und Ethergruppen, Thiolgruppen, Thioethergruppen, Amid- und Estergruppen, Sulfarylgruppen und Sulfoxidgruppen.

Das aromatische System in den Verbindungen der Formel (I) kann ein ein- oder mehrkerniger Aromat sein, der ausschließlich aus Kohlenstoff und Wasserstoff bestehen oder eines oder mehrere Heteroatome aufweisen kann, die aus der Gruppe bestehend aus N, O und S ausgewählt sind.

Vorzugsweise ist das aromatische System ein ein- oder zweikerniger Aromat. Es ist mehr bevorzugt, wenn das aromatische System ein C₅-Heterocyclus oder ein Benzol- oder Naphthalinderivat ist, das eines oder mehrere der erwähnten Heteroatome N, O und S enthalten kann, und Beispiele hierfür sind Benzol und Naphthalin, Aza-, Diaza- und Triazabenzol, Aza-, Diaza- und Triazanaphthalin, Thiophen und Furan.

In der meist bevorzugten Ausführungsform der vorliegenden Erfindung ist das aromatische System in der Formel (I) ausgewählt aus der Gruppe bestehend aus Benzol, Naphthalin, Pyridin, Pyrazin, Pyrimidin, Chinolin, Thiophen und Furan.

Das aromatische System kann sowohl in den bevorzugten als auch den nicht bevorzugten Ausführungsformen einen oder mehrere Substituenten aufweisen, die ähnlich wie die Substituenten R¹ und R² äußerst stark variieren können. Nicht beschränkende Beispiele für solche Substituenten sind Alkane und Alkene, die entweder unsubstituiert sind oder übliche Substituenten aufweisen können, beispielsweise Halogene, Amine, Nitrogruppen, Ether- und Hydroxylgruppen oder Thiol- und Thioethergruppen. Weitere Beispiele für am aromatischen System befindliche Substituenten sind Amino- und Nitrogruppen, Halogene, Hydroxyl- und Ethergruppen, Thiolgruppen, Sulfarylgruppen, Sulfoxidgruppen, Thioethergruppen, Amid- und Estergruppen.

In einer Ausführungsform der vorliegenden Erfindung kann die Synthese der Indolderivate (I) derart durchgeführt werden, daß der als Edukt eingesetzte Alkynylaminoaromat (II) mit einem organischen oder anorganischen Träger verbunden und immobilisiert ist. Diese Träger sind einem Fachmann bekannt und entsprechen den üblichen Trägermaterialien, die beispielsweise für die Festphasen-Peptidsynthese oder zur Fixierung von Übergangsmetallkatalysatorsystemen verwendet werden. Beispiele sind der sogenannte Merrifield-Resin, das 4-(2',4'-Dimethoxyphenyl-Fmoc-aminomethyl)-phenoxyacetamido-norleucyl-Methylbenzhydrylamino-(Divinylbenzol-vernetztes Polystyrol)-Harz, ebenfalls bekannt unter dem Namen Rink-MBHA-Harz, das generell nach Entschützung mittels Abspaltung der 9-Fluorenylmethoxycarbonyl-(Fmoc) Gruppe eingesetzt wird, sowie das unter dem Namen Tritylchlorid-Harz kommerziell erhältliche Trägerharz, gegebenenfalls nach entsprechender Modifizierung.

Diese Träger sind entweder an das aromatische System der Eduktverbindungen (II) gebunden, oder der Substituent R¹ ist mit dem Träger verbunden. Dabei kann der Träger gegebenenfalls über geeignete, sogenannte Spacer-Gruppen, mit der Eduktverbindung verknüpft sein. Nach der Synthese der entsprechenden Indolverbindung wird dann die Bindung zwischen dem Träger und dem Zielmolekül auf die übliche, einem Fachmann bekannte Weise gelöst, beispielsweise durch Spalten der Bindung zwischen Träger und Spacer-Gruppe mit Trifluoracetamid.

In einer weiteren Ausführungsform der vorliegenden Erfindung ist die Synthese von Verbindungen (I) möglich, in denen das aromatische System, das mit der Pyrrolin-Einheit unter Bildung der bicyclischen Indol-Einheit annelliert ist, mit einer weiteren Pyrrolin-Einheit unter Ausbildung eines tricyclischen, bifunktionellen Indols annelliert ist. Dazu wird als Edukt (II) eine Verbindung eingesetzt, die nicht nur jeweils eine Acetylenfunktion und Aminofunktion in vicinaler Stellung aufweist, sondern jeweils zwei dieser Funktionen.

Das erfindungsgemäße Syntheseelement kann auch so erweitert werden, daß nicht nur 2 Pyrrolin-Einheiten an dem erhaltenen Indol vorliegen, sondern 3 oder sogar noch mehr dieser Einheiten. Dazu wird dann eine Eduktverbindung (II) eingesetzt, die dann jeweils 3 oder noch mehr vicinale Acetylen- und Aminofunktionen aufweist.

Die erfindungsgemäße Synthese wird durchgeführt mit einer geeigneten Verbindung von Na, K, Rb oder Cs, die in Verbindung mit einem polaren aprotischen Lösungsmittel mit ionensolvatisierenden Eigenschaften verwendet wird. Durch die Verwendung dieser Lösungsmittel kann die Elektrophilie der Ionen der genannten Metalle so gesteigert werden, daß die erfindungsgemäße Cyclisierungsreaktion unter milden Bedingungen durchgeführt werden kann.

Diese polaren aprotischen, ionensolvatisierenden Lösungsmittel sind einem Fachmann bekannt, und Beispiele sind N-Methylpyrrolidon (NMP), N-Butylpyrrolidon (NBP), Dimethylsulfoxid (DMSO), Sulfolan (Tetrahydrothiophen-1,1-dioxid), Dimethylformamid (DMF), Tetrahydrofuran (THF), Ether, Hexamethylphosphortriamid (HMPT) und N, N'-Dimethylpropylenharnstoff.

Beispiele für geeignete Ether sind aus Ethylenoxid und/oder Propylenoxid erhältliche Ether. Dies können sein Ethylenglykol- und Propylenglykolether, Oligo- bzw. Polyethylenglykol, Oligo- und Polypropylenglykol und Ethylenglykol-Propylenglykol-Copolymere bzw. -Blockcopolymere, wie beispielsweise die unter dem Namen Glyme, Diglyme, Triglyme oder den Namen Pluronic® und Pluriol® (Herkunft: BASF AG) bekannten Verbindungen. Es können auch Ether verwendet werden, die durch Kondensation von Ethylenoxid und/oder Propylenoxid mit mehrwertigen Aminen hergestellt werden, beispielsweise die unter dem Namen Tetronic® bzw. Lutensol® erhältlichen Produkte (Herkunft jeweils: BASF AG).

Von allen diesen oben erwähnten Ethylenglykol- und Propylenglykolethern, die wie etwa im Fall von Pluronic® , Pluriol® und Tetronic® noch freie terminale Hydroxylgruppen aufweisen können, können auch die jeweiligen Ether eingesetzt werden, beispielsweise die Methylether. Weiterhin sind als Ether auch Kronenether und Cryptanden geeignet.

Diese Lösungsmittel können allein, als Gemisch oder als Additiv zu anderen Lösungsmitteln verwendet werden. Dabei ist darauf zu achten, daß sie in einer Menge zugegeben werden, die zum Erreichen der gewünschten Ionensolvatisierung und somit der erforderlichen Reaktivität ausreicht.

Die Verbindungen von Na, K, Rb und Cs, die eingesetzt werden, sind solche, die in den genannten Lösungsmitteln gut dissoziieren und deren Ionen von diesen hinreichend solvatisiert werden. Beispiele für geeignete Verbindungen der zitierten Alkalimetalle sind Hydroxide, Hydride, Alkoholate, Amide und Aminopropylamide.

Besonders geeignete Verbindungen sind Hydroxide und Alkoholate.

Unter den genannten Metallen Na, K, Rb und Cs werden die besten Reaktivitäten bei Verwendung von K und Cs erhalten, deren Einsatz bevorzugt ist. Die erfindungsgemäß verwendeten Alkalimetallverbindungen lassen sich in stöchiometrischen bzw. überstöchiometrischen Mengen, aber auch in katalytischen Mengen einsetzen. Die Mindestmenge an Alkalimetallverbindungen liegt bei Werten von 5 mol-%, es können aber auch Mengen bis 250 mol-% bezüglich des Substrats verwendet werden. Die Reaktionstemperaturen, die bei dem erfindungsgemäßen Verfahren notwendig sind, liegen bei Werten von < 100°C. Vorzugsweise wird das erfindungsgemäße Verfahren bei Temperaturen von ca. 20 bis 60°C, meist bevorzugt von ca. 20 bis 40°C, durchgeführt. Die Reaktionszeiten betragen ca. 2 bis 20 Stunden, vorzugsweise 2 bis 8 Stunden. Die Eduktverbindungen (II) werden nach der sogenannten Sonogashira-Reaktion (siehe K. Sonogashira et al., Synthesis 1980, Seiten 627 ff., K. Sakomoto, Synthesis 1983, Seiten 312 ff., G. C. Fu et al., Angew. Chem. Int. Ed. Engl. 1999 (38), Seiten 2411 ff.) aus den entsprechenden 2-Iod- oder 2-Bromanilinen durch Umsetzung mit 1-Alkinen erhalten. Wird das erfindungsgemäße Verfahren an einem Träger, in Festphase, durchgeführt, dann wird vor der Sonogashira-Kopplung das entsprechende Iod- oder Bromanilin nach einem Fachmann bekannten Methoden an dem Träger fixiert. Die Erfindung wird nun in den nachfolgenden Beispielen erläutert.

### Beispiel 1

### Synthese von 2-Phenylindol mit verschiedenen Alkalimetallverbindungen

Eine Lösung von 1,05 mmol der jeweiligen Alkalimetallverbindung wurde in 4 ml NMP unter Argon gelöst bzw. suspendiert. Anschließend wurden 97 mg (0,5 mmol) 2-Phenylethynylanilin in 1 ml NMP zugegeben. Die Lösung wurde anschließend bei den in Tabelle 1 angegebenen Temperaturen und über die angegebenen Reaktionszeiten heftig gerührt. Anschließend werden 1 ml Wasser und 50 ml Dichlormethan zugegeben und die erhaltene Lösung mit einer gesättigten NaCl-Lösung gewaschen. Nach Trocknen über MgSO₄ und Abfiltrieren des Rückstands wurde die Lösung unter vermindertem Druck eingeengt und der erhaltene Rückstand mit einem CH₂Cl₂/Pentan-Gemisch über eine Silicagel-Kolonne chromatographisch gereinigt. Nach Entfernen des Lösungsmittels wurde das Produkt in reiner Form erhalten.

Die Ergebnisse finden sich in Tabelle 1. Die angegebenen Ausbeuten beziehen sich dabei auf analysenreines Endprodukt.

**Tabelle 1**

| Versuch | Base | Temp. [°C] | Zeit [h] | Ausbeute [%] |
|---|---|---|---|---|
| A | NaH | 60 | 8 | >5 |
| B | NaOEt | 80 | 15 | 66 |
| C | KOt-Bu | 25 | 4 | 79 |
| D | KH | 25 | 5 | 72 |
| E | CsOH | 90 | 5 | 68 |
| F | CsOt-Bu | 25 | 5 | 71 |

### Beispiel 2

Synthese verschiedener Indol- und Azaindolderivate

### Methode A

Zu einer gerührten Lösung von 42 mg (1,05 mmol) KH in 4 ml NMP werden unter Argon 0,5 mmol des jeweiligen Edukts gegeben. Nach 3 bis 12 Stunden bei Raumtemperatur wurde die Reaktionslösung wie in Beispiel 1 angegeben aufgearbeitet. Die Ergebnisse sind in Tabelle 2 wiedergegeben.

### Methode B

Die Reaktion wurde unter A beschrieben durchgeführt, wobei als Alkalimetallverbindung KOt-Bu verwendet wurde. Die Reaktionszeit betrug 4 Stunden bei Raumtemperatur. Die Aufarbeitung geschah wiederum wie in Beispiel 1 beschrieben. Die Ergebnisse finden sich ebenfalls in Tabelle 2.

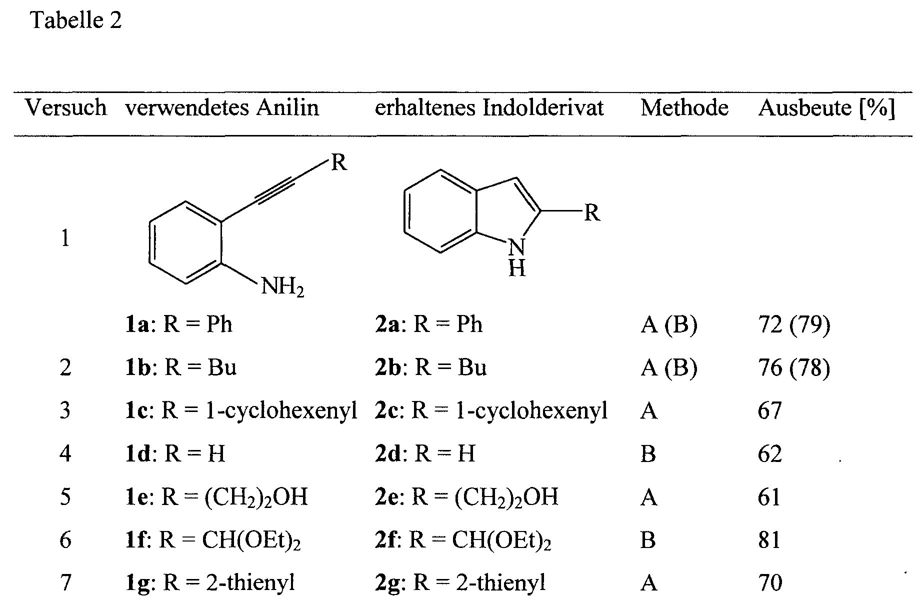

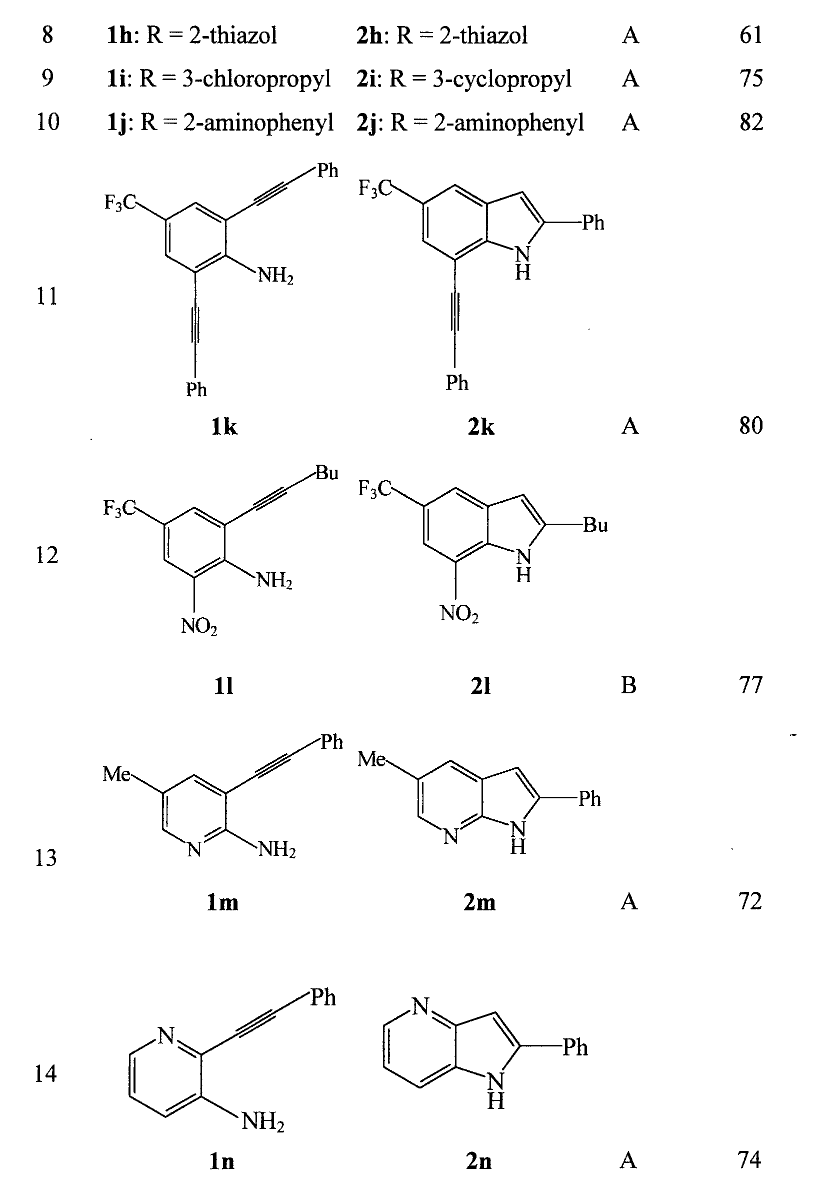

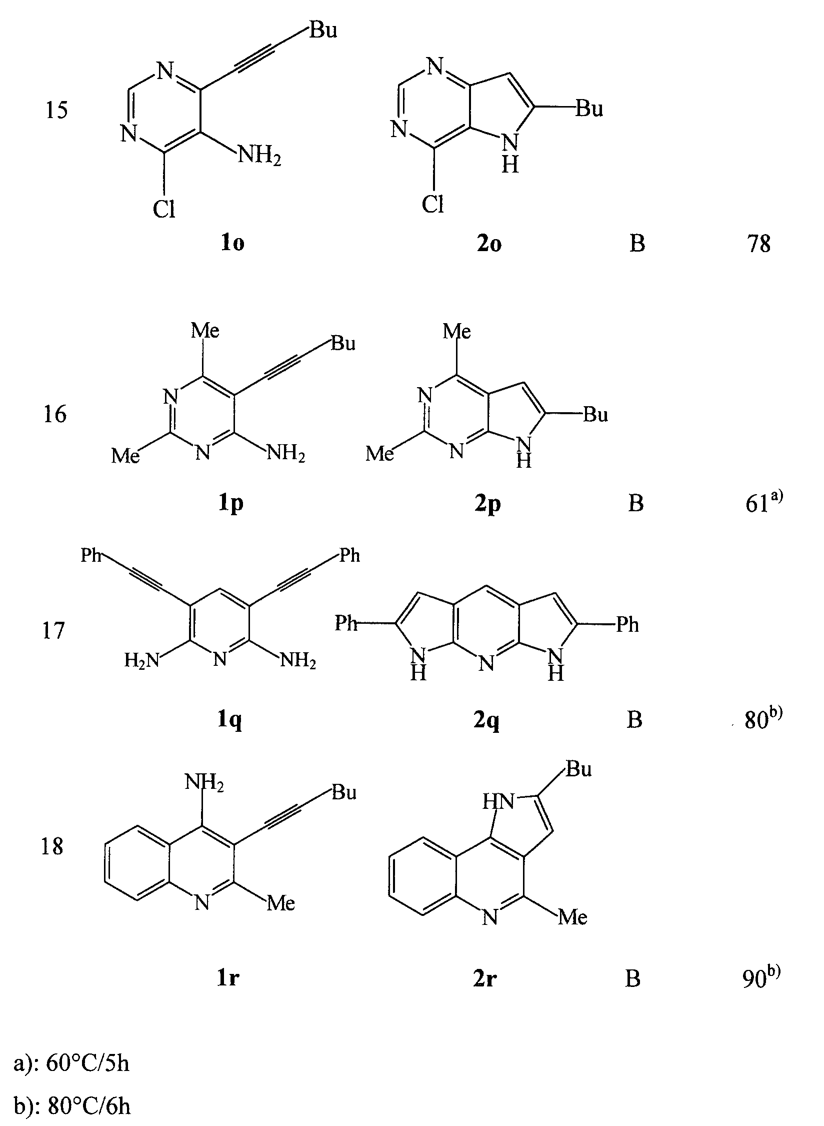

### Beispiel 3

### Festphasensynthese substituierter Indole

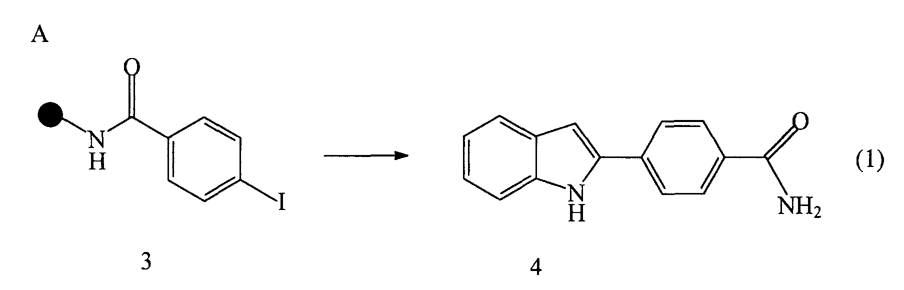

a) Herstellung der Eduktverbindung 3
   Entschütztes Rink-MBHA-Harz (2 g; 0,64 mmol/g erhältlich durch Umsetzung von Rink-MBHA-Harz mit Piperidin) wurde in einem argonbefüllten Schüttlerglas mit Septum für ca. 10 min in Dichlormethan (15 mL) gequellt und anschließend mit 4-Iodbenzoesäure (478 mg; 1,92 mmol und N, N'-Diisopropylcarbodiimid (DIC) (240 mg; 1,92 mmol) versetzt. Die Suspension wurde 20 h bei Raumtemperatur geschüttelt. Das Polymer wurde filtriert und abwechselnd mit Dichlormethan, Methanol, THF und DMF (je ca. 15 mL) gewaschen. Der Waschvorgang wurde dreimal wiederholt. Dann wurde viermal mit Dichlormethan (je ca. 15 mL) gewaschen und das Polymer bei 55°C für ca. 16 h getrocknet.
b) Das erhaltene Produkt wurde anschließend zwei aufeinanderfolgenden Sonogashira-Kupplungen, der erfindungsgemäßen Cyclisierungsreaktion und der Abspaltungsreaktion von Polymerharz unterworfen.

Dazu wurden in der ersten Kupplung polymergebundenes 4-Iodbenzamid (500 mg; 0,25 mmol), Pd(PPh₃)₂Cl₂ (17,4 mg; 0,025 mmol) und CuI (11,8 mg; 0,062 mmol) in einem argonbefüllten Schlenkrohr mit Septum vorgelegt und in Toluol (5 mL) für ca. 10 min gequellt. Dann wurde Diethylamin (5 mL) zugegeben, auf 0°C gekühlt und Trimethylsilylacetylen (245 mg; 2,5 mmol) hinzugegeben. Es wurde 12 h bei Raumtemperatur gerührt, das Polymer dann filtriert und mit DMF, Methanol und THF (je ca. 10 mL) gewaschen. Der Waschvorgang wurde viermal wiederholt. Anschließend wurde viermal mit Dichlormethan (je ca. 10 mL) gewaschen und dann mit TBAF (5 mL; 0,25 M in THF) versetzt und für ca. 15 min geschüttelt. Das Harz wurde wie zuvor beschrieben gewaschen und für ca. 16 h bei 55°C getrocknet.

Die Durchführung der zweiten Kopplung wurde analog der ersten Kopplung durchgeführt, inclusive Waschvorgang und Trocknung.

### Verwendete Mengen:

Harzgebundenes 4-Ethinylbenzamid (400 mg; 0,22 mmol); Pd(PPh₃)₂Cl₂ (15,4 mg; 0,022 mmol); CuI (10,5 mg; 0,055 mmol); Toluol (4 mL); Diethylamin (4 mL); 2-Iodanilin (450 mg; 2,2 mmol).

Das so erhaltene, harzgebundene, aminosubstituierte Diphenylacetylen kann auch in einem Schritt aus dem harzgebundenen 4-Iodbenzamid und 2-Trimethylsilylethinylanilin erhalten werden. Die Durchführung erfolgt völlig analog zur ersten Kopplung inklusive Waschvorgang und Trocknung. Die einzige Modifikation besteht darin, daß hier das terminale Alkin zuvor durch Entschützung mit Tetrabutylammoniumfluorid (TBAF) in THF generiert und nach weitgehender Entfernung des Lösungsmittels im Hochvakuum zugegeben wird.

### Verwendete Mengen:

Harzgebundenes 4-Iodbenzamid (500 mg; 0,25 mmol); Pd(PPh₃)₂Cl₂ (17,5 mg; 0,025 mmol); CuI (11,9 mg; 0,063 mmol); Toluol (5 mL); Diethylamin (5 mL); 2-Trimethylsilylethinylanilin (475 mg; 2,5 mmol); TBAF (11,0 mL, 2,75 mmol).

Das derart erhaltene Zwischenprodukt wurde dann wie folgt zum Indol cyclisiert:

Das polymergebundene Edukt (200 mg; 0,09 mmol) wurde in einem argonbefüllten Schlenkrohr mit Septum vorgelegt und in NMP (4 mL) für ca. 5 min gequellt. Dann wurde auf 0°C gekühlt, und es erfolgte tropfenweise Zugabe einer Lösung von KOtBu (150 mg; 1,35 mmol) in NMP (12 mL). Dann wurde 24 h bei Raumtemperatur gerührt, das Harz filtriert und mit Dichlormethan, THF, Methanol, DMF (je ca. 5 mL) gewaschen. Der Waschvorgang wurde etwa zehn- bis zwölfinal wiederholt. Anschließend wurde viermal mit Dichlormethan (je ca. 5 mL) gewaschen und das Polymer für ca. 16 h bei 55°C getrocknet.
Das so erhaltene Harz wurde dann mit TFA / Dichlormethan 1:1 gespalten.

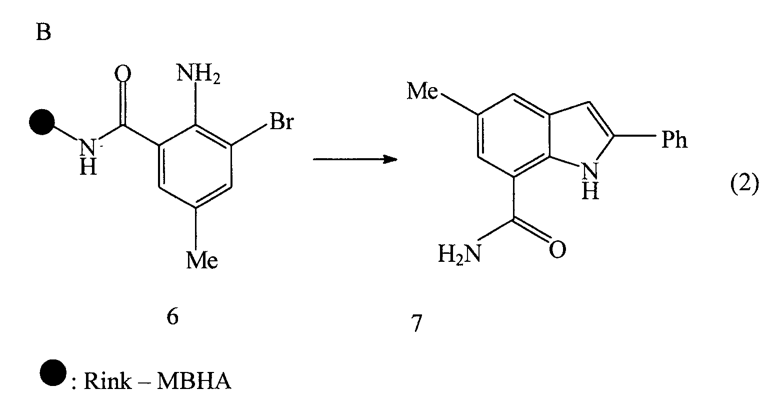

Die Sonogashira-Kopplung wurde wie oben unter A beschrieben, jedoch bei 80°C über 18 h, durchgeführt, als Edukt wurde harzgebundenes 2-Brom-4-methylanilin verwendet. Es wurden 50 mol-% CuI eingesetzt. Die Umsetzung mit KOt-Bu wurden dann analog A durchgeführt, ebenso wie die Abspaltung des Harzes.

## Patentansprüche

1. Verfahren zur Herstellung von Verbindungen des Indoltyps der allgemeinen Formel 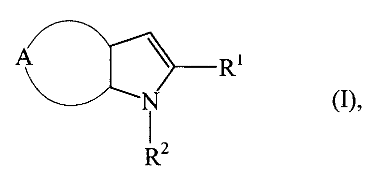 in der A einen Kohlenwasserstoffrest bedeutet, der gemeinsam mit den Kohlenstoffatomen, an die er gebunden ist, ein substituiertes oder unsubstituiertes, ein- oder mehrkerniges aromatisches System darstellt, das eines oder mehrere Heteroatome aus der Gruppe von N, O und S enthalten kann, und
R¹, R² unabhängig voneinander H, einen gesättigten, linearen oder verzweigten aliphatischen C₁-C₂₀-Kohlenwasserstoffrest, einen ungesättigten, linearen oder verzweigten aliphatischen C₂-C₂₀-Kohlenwasserstoffrest, einen gesättigten oder ungesättigten, gegebenenfalls alkylsubstituierten cycloaliphatischen C₃-C₂₀-Kohlenwasserstoffrest oder einen aromatischen C₅-C₂₀-Kohlenwasserstoffrest darstellen, wobei diese Reste eines oder mehrere Heteroatome aus der Gruppe bestehend aus Halogenen, N, P, O, S, Si, Sn und B in ihrem Molekülgerüst enthalten und substituiert oder unsubstituiert sein können,
durch Cyclisierung von Alkynylaminoaromaten der allgemeinen Formel 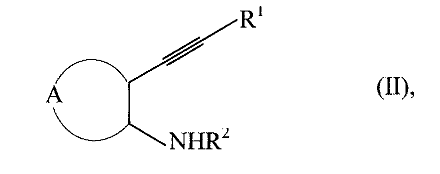 in der R¹ und R² die in Formel (I) angegebene Bedeutung aufweisen, und R¹, R² oder A gegebenenfalls mit einem organischen oder anorganischen Träger verbunden sind,
dadurch gekennzeichnet, daß die Reaktion in einem Polaren aprotischen, ionensolvatisierenden Lösungsmittel in Anwesenheit einer geeigneten Verbindung von Na, K, Rb oder Cs, vorzugsweise K oder Cs, durchgeführt wird.

2. Verfahren nach Anspruch 1, dadurch gekennzeichnet, daß die Substituenten R¹ und R² unabhängig voneinander ausgewählt sind aus der Gruppe bestehend aus H, linearen und verzweigten C₁-C₁₂-Alkylgruppen, linearen und verzweigten C₂-C₁₂-Alkenylgruppen. C₃-C₈-Cycloalkylgruppen, C₃-C₈-Cycloalkenylgruppen, C₅- und C₆-Heterocyclen mit einem oder mehreren Atomen im Ring, die ausgewählt sind aus der Gruppe bestehend aus N, O und S und ein- oder zweikernigen Aromaten mit einem oder mehreren Atomen im Ring, die ausgewählt sind aus N, O und S.

3. Verfahren nach Anspruch 1 oder 2, dadurch gekennzeichnet, daß die für R¹ und R² definierten Kohlenwasserstoffgruppen einen oder mehrere Substituenten ausgewählt aus der Gruppe bestehend aus Amino- und Nitrogruppen, Halogenen, Hydroxyl- und Ethergruppen, Thiolgruppen, Thioethergruppen, Amid- und Estergruppen, Sulfarylgruppen und Sulfoxidgruppen aufweisen.

4. Verfahren nach einem der Ansprüche 1 bis 3, dadurch gekennzeichnet, daß ein Hydrid, Hydroxid, Alkoholat, Amid oder Aminopropylamid von Na, K, Rb oder Cs verwendet wird.

5. Verfahren nach einem der Ansprüche 1 bis 4, dadurch gekennzeichnet, daß als Lösungsmittel N-Methylpyrrolidon, N-Butylpyrrolidon, Dimethylsulfoxid, Sulfolan, Dimethylformamid, Tetrahydrofuran, Hexamethylphosphortriamid N,N'-Dimethylpropylenharnstoff oder ein Ether, insbesondere ein aus Ethylenoxid und/oder Propylenoxid oder aus Ethylenoxid und Propylenoxid und einem mehrwertigen Amin hergestellter Ether, oder ein Gemisch dieser Lösungsmittel verwendet wird.

6. Verfahren nach einem der Ansprüche 1 bis 5, dadurch gekennzeichnet, daß in der Verbindung nach Formel (I) das aromatische System ein substituierter oder unsubstituierter ein- oder zweikerniger Aromat ist, vorzugsweise ein substituierter oder unsubstituierter Aromat, der ausgewählt ist aus der Gruppe bestehend aus C₅-Heterocyclen, Benzol- und Naphthalinderivaten, meist bevorzugt aus der Gruppe bestehend aus Benzol, Naphthalin, Pyridin, Pyrazin, Pyrimidin, Chinolin, Thiophen und Furan.

7. Verfahren nach einem der Ansprüche 1 bis 6, dadurch gekennzeichnet, daß Alkalimetallverbindung in einer Menge von 5 mol-% bis 150 mol-%, bezogen auf das Substrat, eingesetzt wird.

8. Verfahren nach einem der Ansprüche 1 bis 7, dadurch gekennzeichnet, daß das Verfahren bei Temperaturen <100°C, vorzugsweise 20 bis 60°C, meist bevorzugt 20 bis 40°C, durchgeführt wird.

9. Verfahren nach einem der Ansprüche 1 bis 8, dadurch gekennzeichnet, daß die Reaktionszeit 2 bis 20 Stunden, vorzugsweise 2 bis 8 Stunden, beträgt.

10. Verfahren nach einem der Ansprüche 1 bis 9, dadurch gekennzeichnet, daß der als Edukt eingesetzte Alkynylaminoaromat (II) mit einem organischen oder anorganischen Träger, der vorzugsweise ausgewählt ist aus der Gruppe bestehend aus Merrifield-Resin, Rink-MHBA-Resin und Tritylchlorid-Harz, gegebenenfalls nach entsprechender Modifizierung, verbunden und immobilisiert ist, und nach beendeter Synthese die Bindung zwischen Trägermaterial und Zielmolekül auf an sich bekannte Art und Weise gelöst wird.
